# EUROPÄISCHE PATENTSCHRIFT

(11) **EP 3 095 437 B1**
(45) Veröffentlichungstag und Bekanntmachung des Hinweises auf die Patenterteilung: **03.07.2019**
(21) Anmeldenummer: 15168565.8
(22) Anmeldetag: 21.05.2015
(51) Int. Cl.: A61K 8/34, A61K 8/41, A61Q 5/00, A61K 8/44, A61Q 5/12, A61Q 5/06, A61K 8/06, A61Q 5/10

(54) **WÄSSRIGE ZUSAMMENSETZUNG ENTHALTEND OMEGA-AMINOCARBONSÄUREESTER UND FETTALKOHOL**
AQUEOUS COMPOSITION CONTAINING OMEGA AMINOCARBOXYLIC ACID ESTERS AND FATTY ALCOHOL
COMPOSITION AQUEUSE CONTENANT DE L'ACIDE AMINO-CARBOXYLIQUE OMÉGA ET DE L'ALCOOL GRAS

(43) Veröffentlichungstag der Anmeldung: 23.11.2016
(73) Patentinhaber: Evonik Degussa GmbH, 45128 Essen (DE)
(72) Erfinder: Schuch, Dominik, 42781 Haan (DE); Hartung, Christian, 45133 Essen (DE); Wenk, Hans Henning, 45470 Mülheim an der Ruhr (DE); Springer, Oliver, 46485 Wesel (DE); Schwab, Peter, 45133 Essen (DE); Muss, Peter, 45359 Essen (DE)

(56) Entgegenhaltungen:
- EP-A2- 0 928 608
- WO-A1-2011/002746
- WO-A2-2013/150044
- FR-A1- 2 914 852
- US-A1- 2007 213 244

## Beschreibung

### Gebiet der Erfindung

Gegenstand der Erfindung sind wässrige Zusammensetzungen enthaltend ω-Aminocarbonsäureester und Fettalkohol.

### Stand der Technik

Der Einsatz von aminogruppenhaltigen Verbindungen als Konditioniermittel für Haut und Haar in Kosmetika ist seit langem weit verbreitet.
WO2011002746 beispielsweise beschreibt α-Aminosäureester als Konditioniermittel.
US2007213244 offenbart eine Zusammensetzung, umfassend eine N-Langkettenacyl-Aminosäure, ein Salz einer N-Langkettenacyl-Aminosäure oder Mischungen davon; und Hydroxypropylstärkephosphat.
WO2013150044 offenbart kosmetische Zusammensetzungen in Emulsionsform, umfassend Ester einer neutralen Aminosäure, Fettalkohol und ätherisches Öl. Aufgabe der Erfindung war es, neue Zusammensetzungen mit hervorragenden Konditioniereigenschaften bereitzustellen.

### Beschreibung der Erfindung

Überraschenderweise wurde gefunden, dass die im Folgenden beschriebenen Zusammensetzungen herausragende Anwendungseigenschaften aufweisen.

Gegenstand der vorliegenden Erfindung sind daher wässrige Zusammensetzungen enthaltend als Komponente A) ω-Aminocarbonsäureester und als Komponente B) Fettalkohol.
Ein weiterer Gegenstand der vorliegenden Erfindung ist die Verwendung von ω-Aminocarbonsäureestem zur Konditionierung von Haut und keratinischen Fasern, insbesondere Haaren.
Noch ein Gegenstand der vorliegenden Erfindung ist die Verwendung von ω-Aminocarbonsäureestem als Emulgator, insbesondere kationischer Emulgator, in bevorzugt kosmetischen Formulierungen.

Ein Vorteil der vorliegenden Erfindung ist es, dass die erfindungsgemäße Zusammensetzung komplett auf Basis natürlicher Rohstoffe bereitgestellt werden kann.
Noch ein Vorteil der vorliegenden Erfindung ist es, dass der konditionierende Effekt ohne den Einsatz permanenter Quat-Strukturen hervorgerufen werden kann.
Ein weiterer Vorteil ist, dass die eingesetzten ω-Aminocarbonsäureester über eine verbesserte Hydrolysestabilität verfügen.
Noch ein Vorteil der vorliegenden Erfindung ist es, dass die gute Konditionierleistung ohne negativen Einfluss auf die Formulierungsviskosität einhergeht.
Ein weiterer Vorteil ist, dass die eingesetzten ω-Aminocarbonsäureester sich auch in Shampoos einsetzen lassen und dabei konditionierend aktiv sind.
Ein weiterer Vorteil ist, dass die eingesetzten ω-Aminocarbonsäureester gute Bioabbaubarkeit aufweisen.
Noch ein Vorteil der der vorliegenden Erfindung ist es, dass der Glanz der behandelten keratinischen Fasern gesteigert wird.
Ein weiterer Vorteil der vorliegenden Erfindung ist, dass die eingesetzten ω-Aminocarbonsäureester bereits bei geringen Einsatzmengen eine gute Wirkung entfalten.
Ein weiterer Vorteil ist, dass die eingesetzten ω-Aminocarbonsäureester in ökologischer Hinsicht wenig belastend sind.
Noch ein Vorteil ist es, dass die eingesetzten ω-Aminocarbonsäureester auf keratinischen Fasern eine leichtere Ausspülbarkeit zeigen als bisher bekannte quartäre Esterverbindungen.
Noch ein Vorteil der vorliegenden Erfindung ist es, dass die eingesetzten ω-Aminocarbonsäureester nicht auskristallisieren.
Noch ein Vorteil der vorliegenden Erfindung ist es, dass sie Haarfärbemittel vor dem Auswaschen schützt.
Noch ein Vorteil der vorliegenden Erfindung ist es, dass die ω-Aminocarbonsäureester sich leichter formulieren lassen.
Noch ein Vorteil der vorliegenden Erfindung ist es, dass sie die ω-Aminocarbonsäureester Kämmkräfte am nassen und am trockenen Haar reduzieren. Ein weiterer Vorteil ist, dass sich die eingesetzten ω-Aminocarbonsäureester in hohen Aktivstoffgehalten einsetzen lassen.

Noch ein Vorteil der vorliegenden Erfindung ist es, dass die eingesetzten ω-Aminocarbonsäureester als Emulgatoren in O/W-Emulsionen ein hervorragendes Hautgefühl geben.
Noch ein Vorteil der vorliegenden Erfindung ist es, dass sie besonders wirtschaftlich ist.

Unter dem Begriff "wässrig" im Zusammenhang mit der vorliegenden Erfindung ist eine Zusammensetzung zu verstehen, die mindestens 5 Gew.-%, bevorzugt mindestens 30 Gew.-%, insbesondere mindestens 70 Gew.-%, Wasser, bezogen auf die betrachtete Gesamtzusammensetzung enthält.
Der "pH-Wert" im Zusammenhang mit der vorliegenden Erfindung ist definiert als der Wert, welcher für den entsprechenden Stoff bei 25 °C nach fünf Minuten Rühren mit einer gemäß ISO 4319 (1977) kalibrierten pH-Elektrode gemessen wird.
Unter dem Begriff "ω-Aminocarbonsäureester" werden sowohl die Ester als auch deren Salze verstanden. Die Salze liegen im Wässrigen insbesondere an der ω-AminoGruppe protoniert vor.

ω-Aminocarbonsäureester, die in den erfindungsgemäßen Zusammensetzungen enthalten sein können, sind beispielsweise Veresterungsprodukte von ω-Aminocarbonsäuren mit mindestens einem Alkohol, wie zum Beispiel Fettalkohlen und Polyolen, wie zum Beispiel Alkylenglykole, Glycerin, Polyglycerin, oder Mischungen davon. Sind Polyole mit der ω-Aminocarbonsäure verestert, so können weitere Carbonsäuren, z.B. Fettsäuren mit in dem erfindungsgemäß enthaltenem ω-Aminocarbonsäureester eingeestert sein.

Bevorzugt enthaltene ω-Aminocarbonsäureester sind ausgewählt aus der Gruppe umfassend, bevorzugt bestehend aus, ω-Aminocarbonsäureester der allgemeinen Formel 1, der allgemeinen Formel 2 und der allgemeinen Formel 3, 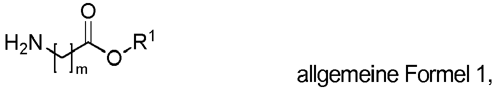 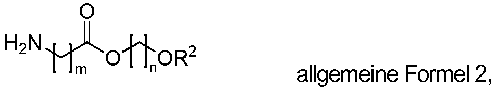 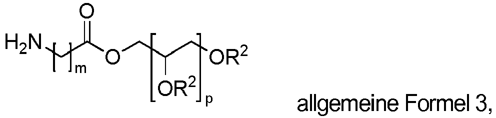 mit
m = 5 bis 21, bevorzugt 7 bis 17, besonders bevorzugt 11,
n = 2 bis 12, bevorzugt 3 bis 6,
p = 1 bis 10, bevorzugt 1 bis 4, besonders bevorzugt 1
R¹ = linearer oder verzweigter, gegebenenfalls ungesättigte Bindungen aufweisender Alkylrest mit 6 bis 22, bevorzugt 8 bis 18, besonders bevorzugt 12 bis 18, Kohlenstoffatomen,
R² = unabhängig voneinander H oder ein linearer oder verzweigter, gegebenenfalls ungesättigte Bindungen aufweisender Acylrest mit 6 bis 22, bevorzugt 8 bis 18, besonders bevorzugt 12 bis 18, Kohlenstoffatomen oder ein ω-Aminoacylrest, insbesondere 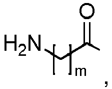 mit m wie oben definiert.

Besonders bevorzugt enthaltene ω-Aminocarbonsäureester sind ausgewählt aus ω-Aminocarbonsäureestem der allgemeinen Formel 1
mit
m = 11 und
R¹ = ausgewählt aus Lauryl, Myristyl, Palmityl, Oleyl und Stearyl,
und ω-Aminocarbonsäureestem der allgemeinen Formel 3
mit
m = 11, p = 1,
R² = ausgewählt aus H, Lauroyl, Myristoyl, Palmitoyl, Oleoyl, Stearoyl und 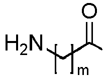 mit m = 11.

Komponente A) ist in den erfindungsgemäßen Zusammensetzung bevorzugt in einer Menge von 0,1 bis 7 Gew.-%, bevorzugt 0,2 bis 5 Gew.-% und insbesondere bevorzugt 0,3 bis 4 Gew.-%, wobei sich die Gew.-% auf die Gesamtzusammensetzung beziehen, enthalten.

Im Wässrigen liegt in Abhängigkeit vom pH-Wert mindestens ein Teil des ω-Aminocarbonsäureesters an der ω-Aminogruppe protoniert vor. Als Gegenion dieses ω-Aminocarbonsäureester-Kations sind in der erfindungsgemäßen Zusammensetzung Anionen enthalten, insbesondere Chlorid, Bromid, lodid, Alkylsulfat, z.B. Methylsulfat, Ethylsulfat, Alkylsulfonat, z.B. Mesylat oder Esylat, Triflat, Tosylat, Phosphat, Sulfat, Hydrogensulfat, Lactat, Glycolat, Acetat und Citrat.

Die erfindungsgemäßen Zusammensetzungen enthalten mindestens einen Fettalkohol (Komponente B).
Als Fettalkohol wird in diesem Zusammenhang bevorzugt ein unverzweigter oder verzweigter Monoalkohol mit einer Alkylgruppe von 8 bis 30 C-Atomen verstanden, der auch ungesättigt sein kann. Bevorzugte Fettalkohole sind Octanol, Decanol, Laurylalkohol, Isolaurylalkohol, Anteisolaurylalkohol, Myristylalkohol, Isomyristylalkohol, Cetylalkohol, Palmoleylalkohol, Stearylalkohol,lsostearylalkohol, Anteisostearylalkohol, Eicosanol, Petroselinylalkohol, Guerbetalkohol, Arachylalkohol, Gadoleylalkohol, Behenylalkohol, Erucylalkohol, Hectacosanol, Octacosanol, und Melissylalkohol sowie Mischungen davon, insbesondere technische Mischungen, vorzugsweise technische Kokos- oder Talgfettalkohole mit 12 bis 18, vorzugsweise mit 16 bis 18 Kohlenstoffatomen, sowie die einfach ungesättigten Fettalkohole, wie Oleylalkohol, Elaidylalkohol, Delta-9-cis-Hexadecenol, Delta-9-Octadecenol, trans-Delta-9-Octadecenol, cis-Delta-11-Octadecenol,trans-10,cis-12-Hexadecadien-1-ol, Octacosa-10,19-dien-1-ol und mehrfach ungesättigte Fettalkohole wie z.B. Linoleylalkohol (9Z, 12Z-Octadecadien-1-ol), Elaidolinoleylalkohol (9E, 12E-Octadecadien-1-ol), Linolenylalkohol (9Z, 12Z, 15Z-Octadecatrien-1-ol), Elaidolinolenylalkohol (9E, 12E, 15-E-Octadecatrien-1-ol), wobei Mischungen von Kokos- oder Talgfettalkohole mit 16 bis 18 Kohlenstoffatomen besonderes bevorzugt sind.
Bevorzugt ist ein Fettalkohol, der einen Schmelzpunkt von größer 25 °C, besonders bevorzugt größer 50 °C, bei 1 bar Druck aufweist.
Der Fettalkohol ist bevorzugt in einer Menge von 0,5 bis 20 Gew.-%, bevorzugt 1 bis 10 Gew.-%, insbesondere 2 bis 7 Gew. %, in der erfindungsgemäßen Zusammensetzung enthalten, wobei sich die Gewichtsprozente auf die Gesamtzusammensetzung beziehen.

Es hat sich als vorteilhaft erwiesen, wenn die erfindungsgemäßen Zusammensetzungen zusätzlich eine Komponente C) Emulgator, insbesondere in einer Menge von 0,1 bis 10 Gew.-%, bevorzugt 0,25 bis 5 Gew.-%, insbesondere 0,5 bis 2,0 Gew.-%, enthalten, wobei sich die Gewichtsprozent auf die Gesamtzusammensetzung beziehen.
Bevorzugt enthaltene Emulgatoren sind nicht-ionische Emulgatoren.

In diesem Zusammenhang bevorzugte Emulgatoren sind ausgewählt aus der Gruppe der Fettalkoholalkoxylate, insbesondere der Fettalkoholethoxylate. Besonders bevorzugt enthaltene Fettalkoholethoxylate sind ausgewählt aus der Gruppe umfassend Polyoxyethylenether des Laurylalkohols, CAS-Nummer 9002-92-0, Macrogollaurylether, z. B. Polyoxyethylen (4) laurylether (Laureth-4, INCI),
Polyoxyethylen (9) laurylether Laureth-9 (INCI),
Polyoxyethylen (23) laurylether Laureth-23 (INCI)
Polyoxyethylenether des Cetylalkohols, CAS-Nummer 9004-95-9, z.B. Polyoxyethylen (2) cetylether Ceteth-2 (INCI),
Polyoxyethylen (10) cetylether Ceteth-10 (INCI)
Polyoxyethylen (20) cetylether Ceteth-20 (INCI)
Polyoxyethylenether des Cetylstearylalkohols, CAS-Nummer 68439-49-6, z.B.
Polyoxyethylen (6) cetylstearylether Ceteareth-6 (INCI)
Polyoxyethylen (20) cetylstearylether Ceteareth-20 (INCI)
Polyoxyethylen (25) cetylstearylether Ceteareth-25 (INCI)
Polyoxyethylenether des Stearylalkohols, CAS-Nummer 9005-00-9, z.B.
Polyoxyethylen (2) stearylether Steareth-2 (INCI)
Polyoxyethylen (10) stearylether Steareth-10 (INCI)
Polyoxyethylen (20) stearylether Steareth-20 (INCI)
Polyoxyethylenether des Oleylalkohols, CAS-Nummer 9004-98-2, z.B.
Polyoxyethylen (2) oleylether Oleth-2 (INCI)
Polyoxyethylen (10) oleylether Oleth-10 (INCI)
Polyoxyethylen (20) oleylether Oleth-20 (INCI)
oder
Polyoxyethylen (10) tridecylether (CAS-Nummer 24938-91-8) und Trideceth-10 (INCI).

Alternativ bevorzugte Emulgatoren sind ausgewählt aus der Gruppe der Polyolester, insbesondere der Glycerinester und Polyglycerinester, insbesondere der Polyglycerinester. Bevorzugt enthaltene (Poly)glycerinester sind dadurch gekennzeichnet, dass sie Partialester sind. Besonders bevorzugte Polyglycerinpartialester sind ausgewählt aus der Gruppe umfassend Polyglycerinpartialester wie in EP-B-0 835 862 beschrieben, die erhältlich sind durch Veresterung eines Polyglyceringemisches mit einem Veresterungsgrad des Polyglycerins zwischen 30 und 75 % und gesättigten oder ungesättigen, linearen oder verzweigten Fettsäuren mit 12 bis 22 C-Atomen und Dimerfettsäuren mit einer mittleren Funktionalität von 2 bis 2,4, Ester der Zitronensäure wie etwa der O/W-Emulgator Glycerylstearatcitrat, (2-Hydroxy-1,2,3-propantricarbonsäure-1,2,3-propantriolmonooctadecanoat, INCI Glyceryl Stearate Citrate, CAS 39175-72-9), der Citronensäureester des Glycerylstearats, u.a. unter der Bezeichnung AXOL C 62 im Handel erhältlich, Glycerylstearatcitrat wie in WO2006034992 und WO2008092676 beschrieben und Glyceryloleatcitrat wie in WO2004112731 beschrieben, ebenso einfache Polyglycerinester, wie beispielsweise Polygylcerin-3 Distearate, Polyglyceryl-10 Stearate, Polyglyceryl-6 Distearate, Mischester aus Polyglycerin und Methylglucose und Stearinsäure, wie beispielsweise Polyglyceryl-3 Methylglucose Distearate und (Poly-)glycerinpartialester mit einer oder mehrerer Carbonsäuren mit 10 bis 24 Kohlenstoffatomen und Resten einer polyfunktionalen Carbonsäure.
Prinzipiell können auch Sorbitan- oder Saccharoseester als Polyolester eingesetzt werden. Eine gängige Kombination ist beispielsweise Sorbitan Stearate & Sucrose Cocoate.

In einer weiteren Alternative bevorzugt enthaltene Emulgatoren sind ausgewählt aus der Gruppe der modifizierten Siloxane, beispielsweise solche, die neben aliphatischen Gruppen auf Basis von alpha-Olefinen auch Polyether tragen. Siloxanbasierte Emulgatoren für Öl-in-Wasser-Emulsionen müssen einen hydrophilen Charakter aufweisen, weshalb es sich in der Regel um reine Polyethersiloxane handelt. Besonders geeignete Beispiele sind relativ hydrophobe Polyethersiloxane wie in EP 1125574 beschrieben, hochmolekulare Polyethersiloxane wie in EP2168564 beschrieben und organomodifizierte Siloxanblockcopolymere wie in WO2009138306 beschrieben. Bevorzugt enthaltene modifizierte Siloxane sind dadurch gekennzeichnet, dass sie einen HLB-Wert >8 aufweisen. Besonders bevorzugte modifizierte Siloxane sind ausgewählt aus der Gruppe umfassend Bis-PEG/PPG-16/16 Dimethicone, PEG/PPG-16/16 Dimethicone, Bis-PEG/PPG-20/5 PEG/PPG-20/5 Dimethicone und Methoxy PEG/PPG-25/4 Dimethicone.
Vorgenannte Emulgatoren ergeben im Zusammenhang mit der vorliegenden Erfindung besonders lagerstabile Formulierungen.

Bevorzugte erfindungsgemäße Zusammensetzungen sind Emulsionen, bevorzugt Öl in Wasser Emulsionen, besonders bevorzugt Emulsionen, bei denen die Ölphase bei 25 °C fest ist, somit liegt bei 25 °C eine Öl-in-Wasser Suspension vor.

Die erfindungsgemäßen Zusammensetzungen weisen vorteilhaft bei 25 °C einen pH-Wert von 1 bis 6,9, besonders bevorzugt von 2 bis 6,5 insbesondere von 2,5 bis 6 auf. Es hat sich als vorteilhaft erwiesen, wenn die erfindungsgemäßen Zusammensetzungen zusätzlich eine Komponente D) Tensid, insbesondere in einer Menge von 0,1 bis 10 Gew.-%, bevorzugt 0,25 bis 5 Gew.-%, insbesondere 0,5 bis 2,0 Gew.-%, enthalten, wobei sich die Gewichtsprozent auf die Gesamtzusammensetzung beziehen.
Unter dem Begriff "Tensid" im Zusammenhang mit der vorliegenden Erfindung werden organische Substanzen mit grenzflächenaktiven Eigenschaften verstanden, die die Fähigkeit besitzen, die Oberflächenspannung von Wasser bei 20°C und bei einer Konzentration von 0,5 Gew.-% bezogen auf die Gesamtzusammensetzung auf unter 45 mN/m zu verringern. Die Oberflächenspannung wird hier durch die Ringmethode nach DuNoüy bei 25°C bestimmt.

Bei den Tensiden handelt es sich insbesondere um nicht-ionische Tenside, anionische Tenside, kationische Tenside und amphotere (zwitterionische) Tenside.

Es sind erfindungsgemäße Zusammensetzungen bevorzugt, die dadurch gekennzeichnet sind, dass das Tensid ausgewählt ist aus der Gruppe umfassend, bevorzugt bestehend aus:
anionische Tenside, kationische Tenside und amphotere Tenside, wobei anionische Tenside und kationische Tenside besonders bevorzugt sind.

Enthält die erfindungsgemäße Zusammensetzung ein anionisches Tensid, sind insbesondere erfindungsgemäße Zusammensetzungen bevorzugt, die dadurch gekennzeichnet sind, dass das anionische Tensid ausgewählt ist aus der Gruppe umfassend, bevorzugt bestehend aus:
Alkylsulfate in Form ihrer Alkali, Ammonium- oder Alkanolammoniumsalze, Alkylethersulfate,
Alkylphosphate in Form ihrer Alkali, Ammonium- oder Alkanolammoniumsalze, Alkylethercarboxylate in Form ihrer Alkali- oder Ammoniumsalze,
Acylsarkosinate in Form ihrer Alkali- oder Ammoniumsalze,
Sulfosuccinate in Form ihrer Alkali- oder Ammoniumsalze und Acylglutamate in Form ihrer Alkali- oder Ammoniumsalze,
wobei Alkylsulfate und Alkylethersulfate besonders bevorzugt sind.

Enthält die erfindungsgemäße Zusammensetzung ein kationisches Tensid, sind insbesondere erfindungsgemäße Zusammensetzungen bevorzugt, die dadurch gekennzeichnet sind, dass das kationische Tensid ausgewählt ist aus der Gruppe der quarternären Ammoniumverbiundungen, bevorzugt bestehend aus Alkyltrimethylammoniumverbindungen, wobei Palmitamidopropyltrimonium Chloride besonders bevorzugt ist.

Enthält die erfindungsgemäße Zusammensetzung ein amphoteres Tensid, sind insbesondere erfindungsgemäße Zusammensetzungen bevorzugt, die dadurch gekennzeichnet sind, dass das amphotere Tensid ausgewählt ist aus der Gruppe umfassend, bevorzugt bestehend aus:
Betaine, Amphoacetate und Amphopropionate,
N-Alkyl-N,N-dimethylammoniumglycinate, beispielsweise das Kokosalkyldimethylammoniumglycinat,
N-Acylaminopropyl-N,N-dimethylammoniumglycinate, beispielsweise das Kokosacylaminopropyldimethylammoniumglycinat,
2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat,
Verbindungen, die außer einer C8/18-Alkyl- oder -Acylgruppe im Molekül mindestens eine freie Aminogruppe und mindestens eine -COOH- oder -SO₃H- Gruppe enthalten und zur Ausbildung innerer Salze befähigt sind, wie zum Beispiel N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe,
N-Kokosalkylaminopropionat, Kokosacylaminoethylaminopropionat und C12/18-Acylsarcosin,
wobei N-Acylaminopropyl-N,N-dimethylammoniumglycinate besonders bevorzugt sind.

Die erfindungsgemäßen Zusammensetzungen können z.B. mindestens eine weitere, zusätzliche Komponente enthalten, ausgewählt aus der Gruppe der
Emollients,
Co-Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.
Bezüglich weiterer fakultativer Komponenten sowie der eingesetzten Mengen dieser Komponenten wird ausdrücklich auf die dem Fachmann bekannten einschlägigen Handbücher, z. B. K. Schrader, "Grundlagen und Rezepturen der Kosmetika", 2. Auflage, Seite 329 bis 341, Hüthig Buch Verlag Heidelberg, verwiesen.
Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Die ω-Aminocarbonsäureester sowie die ω-Aminocarbonsäureester, die in den erfindungsgemäßen Zusammensetzungen bevorzugt enthalten sind, sowie die erfindungsgemäßen Zusammensetzungen lassen sich erfindungsgemäß für die kosmetische Behandlung von Haut oder keratinischer Fasern, insbesondere für die Haarbehandlung, verwenden.

In diesem Zusammenhang werden bevorzugt solche Verbindungen der allgemeinen Formel 1, 2 und 3 bevorzugt verwendet, die oben als bevorzugt in den erfindungsgemäßen Zusammensetzungen enthalten beschrieben werden.
Die erfindungsgemäße Verwendung führt zur Verbesserung der Konditionierung, des Glanzes, der Flexibilität, der Spannkraft, und/oder der Kämmbarkeit sowie einer Reduzierung der Bruchwahrscheinlichkeit der behandelten Faser und außerdem reduziert sie die antistatischen Kräfte zwischen den Fasern.
Noch ein Gegenstand der vorliegenden Erfindung ist die Verwendung von ω-Aminocarbonsäureestem, sowie den ω-Aminocarbonsäureestem, die in den erfindungsgemäßen Zusammensetzungen bevorzugt enthalten sind als Emulgator, insbesondere kationischem Emulgator, in bevorzugt kosmetischen Formulierungen, insbesondere unter Erhalt von O/W-Emulsionen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### 1: Herstellung erfindungsgemäßer ω-Aminocarbonsäureester:

### 1.1. Herstellung von 12-Aminolaurinsäurelaurylester MU4312:

Unter Stickstoffatmosphäre wurde ein Gemisch aus 52,6 g 12-Aminolaurinsäure (1 Moläquiv.) und 93,9 g Laurylalkohol (2 Moläquiv.) bei 90 °C unter Rühren mit einer Lösung von 24,9 g Methansulfonsäure (1,05 Moläquiv.) in 10,3 g Wasser versetzt. Danach wurde das Reaktionsgemisch 10 Stunden bei 140 °C gerührt und kontinuierlich Wasser abdestilliert. Nach Abkühlung auf Raumtemperatur lag das Reaktionsprodukt als gelblich bis bräunlich gefärbter Feststoff vor.

### 1.2. Herstellung von 12-Aminolaurinsäurepalmitylester MU4343:

Unter Stickstoffatmosphäre wurde ein Gemisch aus 48,3 g 12-Aminolaurinsäure (1 Moläquiv.) und 108,8 g Palmitylalkohol (2 Moläquiv.) bei 90 °C unter Rühren mit einer Lösung von 22,9 g Methansulfonsäure (1,05 Moläquiv.) in 9,5 g Wasser versetzt. Danach wurde das Reaktionsgemisch 10 Stunden bei 140 °C gerührt und kontinuierlich Wasser abdestilliert. Nach Abkühlung auf Raumtemperatur lag das Reaktionsprodukt als gelblich bis bräunlich gefärbter Feststoff vor.

### 1.3. Herstellung von 12-Aminolaurinsäurestearylester MU4342:

Unter Stickstoffatmosphäre wurde ein Gemisch aus 45,2 g 12-Aminolaurinsäure (1 Moläquiv.) und 113,5 g Stearylalkohol (2 Moläquiv.) bei 90 °C unter Rühren mit einer Lösung von 21,4 g Methansulfonsäure (1,05 Moläquiv.) in 8,8 g Wasser versetzt. Danach wurde das Reaktionsgemisch 10 Stunden bei 140 °C gerührt und kontinuierlich Wasser abdestilliert. Nach Abkühlung auf Raumtemperatur lag das Reaktionsprodukt als gelblich bis bräunlich gefärbter Feststoff vor.

### 1.4. Herstellung von 12-Aminolaurinsäureoleylester MU4313:

Unter Stickstoffatmosphäre wurde ein Gemisch aus 45,5 g 12-Aminolaurinsäure (1 Moläquiv.) und 112,9 g Oleylalkohol (2 Moläquiv.) bei 90 °C unter Rühren mit einer Lösung von 21,5 g Methansulfonsäure (1,05 Moläquiv.) in 8,9 g Wasser versetzt. Danach wurde das Reaktionsgemisch 10 Stunden bei 140 °C gerührt und kontinuierlich Wasser abdestilliert. Nach Abkühlung auf Raumtemperatur lag das Reaktionsprodukt als gelblich bis bräunlich gefärbter Feststoff vor.

### 1.5. Herstellung von 12-Aminolaurinsäureglyceryllauratester AZMU370:

Unter Stickstoffatmosphäre wurde ein Gemisch aus 65,5 g 12-Aminolaurinsäure (1 Moläquiv.) und 83,5 g Glyceryllaurat (1,1 Moläquiv.) bei 90 °C unter Rühren mit einer Lösung von 31,0 g Methansulfonsäure (1,05 Moläquiv.) in 12,8 g Wasser versetzt. Danach wurde das Reaktionsgemisch 10 Stunden bei 140 °C gerührt und kontinuierlich Wasser abdestilliert. Nach Abkühlung auf Raumtemperatur lag das Reaktionsprodukt als gelblich bis bräunlich gefärbter Feststoff vor.

### 1.6. Herstellung von 12-Aminolaurinester MU4346 mit 1,3-Propandiol:

Unter Stickstoffatmosphäre wurde ein Gemisch aus 98,1 g 12-Aminolaurinsäure (1 Moläquiv.) und 35,4 g 1,3-Propandiol (2 Moläquiv.) bei 90 °C unter Rühren mit einer Lösung von 46,5 g Methansulfonsäure (1,05 Moläquiv.) in 19,9 g Wasser versetzt. Danach wurde das Reaktionsgemisch 10 Stunden bei 140 °C gerührt und kontinuierlich Wasser abdestilliert. Nach Abkühlung auf Raumtemperatur lag das Reaktionsprodukt als gelblich bis bräunlich gefärbter Feststoff vor.

### 1.7. Herstellung von 12-Aminolaurinsäureglycerylester MU4347:

Unter Stickstoffatmosphäre wurde ein Gemisch aus 94,7 g 12-Aminolaurinsäure (1 Moläquiv.) und 40,5 g Glycerin (2 Moläquiv.) bei 90 °C unter Rühren mit einer Lösung von 44,8 g Methansulfonsäure (1,05 Moläquiv.) in 30,4 g Wasser versetzt. Danach wurde das Reaktionsgemisch 10 Stunden bei 140 °C gerührt und kontinuierlich Wasser abdestilliert. Nach Abkühlung auf Raumtemperatur lag das Reaktionsprodukt als gelblich bis bräunlich gefärbter Feststoff vor.

### 2: Nicht-erfindungsgemäße Vergleichsbeispiele:

### 2.1. Behentrimonium Chloride:

VARISOFT® BT 85 (INCI: Behentrimonium Chloride. Evonik Industries AG), 85%ig mit 15% Isopropanol.

### 2.2. Brassicyl Isoleucinate Esylate (and) Brassica Alcohol:

Emulsense HC (INCI: Brassicyl Isoleucinate Esylate (and) Brassica Alcohol. Inolex), ca. 25%ig kationisch in ca. 75% Brassica Alcohol.

### 3: Anwendungsbeispiele: Austestung der Konditionierung von Haar mittels Sensoriktests in einer Haarspülung:

Für die anwendungstechnische Beurteilung der Konditionierung von Haar wurden die erfindungsgemäßen Verbindungen aus den Synthesebeispielen 1.1, 1.4 und 1.5 sowie die kommerziell erhältlichen Produkte 2.1 und 2.2 in einer einfachen kosmetischen Haarspülungs-Formulierung eingesetzt und verglichen.

Die anwendungstechnischen Eigenschaften beim Einsatz in Haarspülungen wurden in den folgenden Rezepturen überprüft:

| **Formulierungsbeispiele** | **0a** | **1a** | **2a** | **3a** | **V4a** | **V5a** |
|---|---|---|---|---|---|---|
| TEGINACID® C, Evonik Industries (INCI: Ceteareth-25) | 0,5% | 0,5% | 0,5% | 0,5% | 0,5% | 0,5% |
| TEGO® Alkanol 16, Evonik Industries (INCI: Cetyl Alcohol) | 5,0% | 5,0% | 5,0% | 5,0% | 5,0% | 5,0% |
| Neolone PE, The Dow Chemical Company (INCI: Phenoxyethanol; Methylisothiazolinone) | 0,45% | 0,45% | 0,45% | 0,45% | 0,45% | 0,45% |
| Wasser, demineralisiert | ad. 100,0% | | | | | |
| Natronlauge (10% in Wasser) | ad. pH 4,3 ± 0,3 | | | | | |
| Synthesebeispiel 1.1, 12-Aminolaurinsäurelaurylester (erfindungsgemäß) | | 1,0% | | | | |
| Synthesebeispiel 1.4, 12-Aminolaurinsäureoleylester (erfindungsgemäß) | | | 1,0% | | | |
| Synthesebeispiel 1.5, 12-Aminolaurinsäureglyceryllauratester (erfindungsgemäß) | | | | 1,0% | | |
| VARISOFT® BT 85 (INCI: Behentrimonium Chloride) 85%ig in Isopropanol (nicht erfindungsgemäß) | | | | | 1,2% | |
| Emulsense HC (INCI: Brassicyl Isoleucinate Esylate (and) Brassica Alcohol), 25%ig in Brassica Alcohol. (nicht erfindungsgemäß) | | | | | | 4,0% |

Die Herstellung der Haarspülungen erfolgte dabei wie im Allgemeinen üblich, indem die Ölphase aus Fettalkohol (Cetyl Alcohol), Emulgator (Ceteareth-25) und den erfindungsgemäßen bzw. nicht-erfindungsgemäßen organischen Konditioniermitteln bis zur Schmelze (∼75 °C) aufgeheizt wurde, und diese Ölphase dann der Wasserphase (Wasser) bei 75 °C unter Rühren zugegeben wurde. Es wurde für 30 Sekunden homogenisiert und sodann unter leichtem Rühren langsam abgekühlt. Die Einstellung des pH-Wertes erfolgte danach mit Natronlauge.
Für das Vergleichsprodukt 2.2 musste jedoch abweichend sehr umständlich erst die Wasserphase vor Homogenisierung auf pH -12 eingestellt werden (entweder mit NaOH-Lösung oder wie in WO2011002746 beschrieben mit L-Arginin), da ansonsten später bei der pH-Wert Einstellung eine inhomogene Emulsion entstand. D.h. je nach Zusammensetzung der Formulierung muss somit meist zweimal der pH-Wert eingestellt werden bei Vergleichsprodukt 2.2, bzw. es muss umständlich ausgetestet werden, welchen pH-Wert man bei der Wasserphase einstellen muss, um am Schluss auf den beabsichtigten pH zu kommen. Trotz des hohen kationischen Aktivstoffgehalts von 100% lassen sich somit die erfindungsgemäßen Produkte deutlich besser verarbeiten als der Aminosäureester Beispiel 2.2 des Standes der Technik, da diese umständliche pH-Wert Einstellung bei den erfindungsgemäßen Produkten nicht nötig ist und keine inhomogenen Emulsionen gebildet werden.

Für die anwendungstechnische Beurteilung wurden Haartressen, die für sensorische Tests verwendet werden, durch eine Bleichbehandlung standardisiert vorgeschädigt. Dazu wurden friseurübliche Produkte verwendet. Der Testablauf, die verwendeten Basismaterialien sowie die Details der Beurteilungskriterien werden in DE 103 27 871 beschrieben.

Die Vorbehandlung der Haare erfolgte durch ein Shampoo, welches keine Konditioniermittel enthält.

Standardisierte Behandlung von vorgeschädigten Haartressen mit konditionierenden Formulierungen:
Die, wie oben beschrieben, vorgeschädigten Haartressen wurden wie folgt mit der oben beschriebenen konditionierenden Spülung behandelt:
Die Haartressen wurden unter fließendem, warmem Wasser benetzt. Das überschüssige Wasser wurde leicht von Hand ausgedrückt, dann wird das nicht konditionierende Shampoo aufgebracht und sanft im Haar eingearbeitet (1 ml/Haartresse (2 g)). Nach einer Verweilzeit von 1 min wurde das Haar für 1 min gespült. Direkt im Anschluss wurde die zu testende Spülung aufgebracht und sanft im Haar eingearbeitet (1 ml/Haartresse (2 g)). Nach einer Verweilzeit von 1 min wurde das Haar für 1 min mit Wasser gespült.

### Beurteilungskriterien:

Die sensorischen Bewertungen der Haartressen erfolgten nach Noten, die auf einer Skala von 1 bis 5 vergeben wurden, wobei 1 die schlechteste und 5 die beste Bewertung ist. Die einzelnen Testkriterien erhielten jeweils eine eigene Bewertung. Die Testkriterien sind: Nasskämmbarkeit, Nassgriff, Ausspülbarkeit, Trockenkämmbarkeit, Trockengriff, Aussehen/Glanz.

In der folgenden Tabelle wurden die Ergebnisse der sensorischen Beurteilung der wie oben beschrieben durchgeführten Behandlung der Haarsträhnchen mit den erfindungsgemäßen Formulierungen 1a, 2a und 3a, den Vergleichsformulierung V4a und V5a sowie der Kontrollformulierung 0a (Placebo ohne Testsubstanz) verglichen.

| | Nasskämmbarkeit | Nassgriff | Ausspülbarkeit | Trockenkämmbarkeit | Trockengriff | Glanz |
|---|---|---|---|---|---|---|
| Kontrollformulierung 0a | 1,9 | 1,9 | 4,0 | 3,5 | 3,6 | 2,0 |
| Erfindungsgemäße Formulierung 1a | 4,1 | 4,0 | 3,5 | 4,1 | 4,1 | 3,5 |
| Erfindungsgemäße Formulierung 2a | 4,0 | 4,0 | 3,3 | 4,3 | 4,3 | 3,6 |
| Erfindungsgemäße Formulierung 3a | 4,0 | 4,0 | 3,2 | 4,4 | 4,3 | 3,6 |
| Vergleichsformulierung V4a (nicht erfindungsgemäß) | 3,8 | 3,7 | 2,8 | 4,3 | 4,2 | 3,3 |
| Vergleichsformulierung V5a (nicht erfindungsgemäß) | 3,9 | 3,6 | 2,6 | 4,3 | 4,3 | 3,2 |

Die erfindungsgemäßen Formulierungen 1a, 2a und 3a mit den erfindungsgemäßen Verbindungen aus den Synthesebeispielen 1.1, 1.4 und 1.5 zeigten in der sensorischen Beurteilung gute kosmetische Bewertungen. Die Kontrollformulierung 0a ohne kationischem Aktivstoff wurde durch die Zugabe von 1,0% Aktivstoff signifikant verbessert. Dabei wurden die bereits sehr guten Eigenschaften der Vergleichsformulierungen V4a und V5a v.a. bzgl. Nassgriff durch die erfindungsgemäßen Formulierungen noch weiter gesteigert. Die Nass- und Trockenkämmbarkeit sowie der Trockengriff sind für alle Produkte auf ähnlich hohem Niveau. Auffallend ist die signifikant bessere Bewertung der erfindungsgemäßen Formulierungen 1a, 2a und 3a bei Glanz des Haares und v.a. auch bei der Ausspülbarkeit. Die Leichtigkeit der Ausspülbarkeit der Konditionierformulierungen mit den erfindungsgemäßen Verbindungen ist besonders überraschend im Vergleich zu den Vergleichsformulierungen V4a und V5a. Dies kann bei der Anwendung ein großer Vorteil (zeittechnisch und ökologisch) sein, da der Konsument schon nach einer kürzeren Ausspülzeit mit Wasser das Gefühl hat, das das Produkt ausgewaschen ist.

### 4: Weitere Formulierungsbeispiele:

Die in den nachfolgenden Tabellen angegebenen Formulierungsbeispiele zeigen exemplarische Vertreter einer Vielzahl von möglichen erfindungsgemäßen Zusammensetzungen.
Wenn nicht anders angegeben, handelt es sich bei den Angaben in den folgenden Tabellen um Angaben in Gew.-%.

**Formulierungsbeispiel 6) Rinse-Off Conditioner**

| | |
|---|---|
| Water | ad. 100,00% |
| VARISOFT® BT 85, Evonik Industries (INCI: Behentrimonium Chloride) | 0,50% |
| Synthesebeispiel 1.1 | 1,50% |
| TEGO® Alkanol 1618, Evonik Industries (INCI: Cetearyl Alcohol) | 5,00% |
| Sodium Hydroxide (10% in water) | ad. pH 5.0 |
| Preservative, Perfume | q.s. |

**Formulierungsbeispiel 7) Rinse-Off Conditioner**

| | |
|---|---|
| Water | ad. 100,00% |
| Synthesebeispiel 1.1 | 1,50% |
| TEGO® Alkanol 1618, Evonik Industries (INCI: Cetearyl Alcohol) | 5,00% |
| Laurylalkohol, Aldrich | 0,50% |
| Glycerin | 1,00% |
| Sodium Hydroxide (10% in water) | ad. pH 4,5 |
| Preservative, Perfume | q.s. |

**Formulierungsbeispiel 8) Rinse-Off Conditioner**

| | |
|---|---|
| Water | ad. 100,00% |
| Synthesebeispiel 1.2 | 1,50% |
| TEGO® Alkanol 1618, Evonik Industries (INCI: Cetearyl Alcohol) | 4,00% |
| Jaguar C-162, Solvay (INCI: Hydroxypropyl Guar Hydroxypropyltrimonium Chloride) | 0,15% |
| Glycerin | 1,00% |
| Sodium Hydroxide (10% in water) | ad. pH 4,8 |
| Preservative, Perfume | q.s. |

**Formulierungsbeispiel 9) Rinse-Off Conditioner**

| | |
|---|---|
| Water | ad. 100,00% |
| Synthesebeispiel 1.1 | 1,50% |
| Synthesebeispiel 1.3 | 0,50% |
| TEGO® Alkanol 1618, Evonik Industries (INCI: Cetearyl Alcohol) | 5,00% |
| Glycerin | 1,50% |
| Sodium Hydroxide (10% in water) | ad. pH 4,3 |
| Preservative, Perfume | q.s. |

**Formulierungsbeispiel 10) Rinse-Off Conditioner**

| | |
|---|---|
| Water | ad. 100,00% |
| Synthesebeispiel 1.5 | 1,50% |
| TEGO® Care PSC 3, Evonik Industries (INCI: Polyglyceryl-3 Dicitrate/Stearate) | 0,50% |
| TEGO® Alkanol 1618, Evonik Industries (INCI: Cetearyl Alcohol) | 5,00% |
| Glycerin | 0,50% |
| Sodium Hydroxide (10% in water) | ad. pH 4,5 |
| Preservative, Perfume | q.s. |

**Formulierungsbeispiel 11) Rinse-Off Conditioner**

| | |
|---|---|
| Water | ad. 100,00% |
| VARISOFT® EQ 100, Evonik Industries (INCI: Quaternium-98 (proposed)) | 1,00% |
| Synthesebeispiel 1.4 | 2,00% |
| TEGO® Care PSC 3, Evonik Industries (INCI: Polyglyceryl-3 Dicitrate/Stearate) | 0,80% |
| TEGO® Alkanol 1618, Evonik Industries (INCI: Cetearyl Alcohol) | 6,50% |
| Sodium Hydroxide (10% in water) | ad. pH 4,8 |
| Preservative, Perfume | q.s. |

**Formulierungsbeispiel 12) Rinse-Off Conditioner**

| | |
|---|---|
| Water | ad. 100,00% |
| ABIL® Quat 3272, Evonik Industries (INCI: Quaternium-80) | 0,50% |
| Synthesebeispiel 1.2 | 2,00% |
| TEGO® Alkanol 1618, Evonik Industries (INCI: Cetearyl Alcohol) | 4,00% |
| Sodium Hydroxide (10% in water) | ad. pH 4,5 |
| Preservative, Perfume | q.s. |

**Formulierungsbeispiel 13) Rinse-Off Conditioner**

| | |
|---|---|
| TEGINACID® C, Evonik Industries (INCI: Ceteareth-25) | 0,50% |
| TEGO® Alkanol 16, Evonik Industries (INCI: Cetyl Alcohol) | 2,00% |
| ABIL® Quat 3272, Evonik Industries (INCI: Quaternium-80) | 0,50% |
| TEGO® Amid S 18, Evonik Industries (INCI: Stearamidopropyl Dimethylamine) | 1,00% |
| Synthesebeispiel 1.3 | 1,50% |
| Propylene Glycol | 1,00% |
| Citric Acid Monohydrate | ad. pH 3,9 |
| Water | ad. 100,00% |
| Preservative, Perfume | q.s. |

**Formulierungsbeispiel 14) Rinse-Off Conditioner**

| | |
|---|---|
| TEGINACID® C, Evonik Industries (INCI: Ceteareth-25) | 0,50% |
| TEGO® Alkanol 16, Evonik Industries (INCI: Cetyl Alcohol) | 5,00% |
| TEGOSOFT® DEC, Evonik Industries (INCI: Diethylhexyl Carbonate) | 0,50% |
| Synthesebeispiel 1.5 | 1,00% |
| Water | ad. 100,00% |
| TEGO® Cosmo C 100, Evonik Industries (INCI: Creatine) | 0,20% |
| Propylene Glycol | 1,00% |
| Sodium Hydroxide (10% in water) | ad. pH 4,8 |
| Preservative, Perfume | q.s. |

**Formulierungsbeispiel 15) Rinse-Off Conditioner**

| | |
|---|---|
| Water | ad. 100,00% |
| VARISOFT® 300, Evonik Industries (INCI: Cetrimonium Chloride) | 2,00% |
| VARISOFT® BT 85, Evonik Industries (INCI: Behentrimonium Chloride) | 1,00% |
| ABIL® OSW 5, Evonik Industries (INCI: Cyclopentasiloxane; Dimethiconol) | 0,70% |
| Synthesebeispiel 1.1 | 1,30% |
| ABIL® Soft AF 300, Evonik Industries (INCI: Aminopropyl Dimethicone) | 1,00% |
| TEGO® Alkanol 1618, Evonik Industries (INCI: Cetearyl Alcohol) | 5,00% |
| Citric Acid Monohydrate | ad. pH 3,8 |
| Preservative, Perfume | q.s. |

**Formulierungsbeispiel 16) Rinse-Off Conditioner**

| | |
|---|---|
| Water | ad. 100,00% |
| ABIL® Soft AF 100, Evonik Industries (INCI: Methoxy PEG/PPG-7/3 Aminopropyl Dimethicone) | 1,00% |
| Synthesebeispiel 1.2 | 2,80% |
| TEGO® Alkanol 1618, Evonik Industries (INCI: Cetearyl Alcohol) | 5,00% |
| Glycerin | 1,00% |
| Sodium Hydroxide (10% in water) | ad. pH 4,6 |
| Preservative, Perfume | q.s. |

**Formulierungsbeispiel 17) Rinse-Off Conditioner**

| | |
|---|---|
| Water | ad. 100,00% |
| VARISOFT® BT 85, Evonik Industries (INCI: Behentrimonium Chloride) | 1,00% |
| SF 1708, Momentive (INCI: Amodimethicone) | 1,50% |
| Synthesebeispiel 1.2 | 1,20% |
| TEGO® Alkanol 1618, Evonik Industries (INCI: Cetearyl Alcohol) | 5,00% |
| Sodium Hydroxide (10% in water) | ad. pH 4,8 |
| Preservative, Perfume | q.s. |

**Formulierungsbeispiel 18) Leave-In Conditioner Spray**

| | |
|---|---|
| Lactic Acid, 80% | 0,20% |
| Water | ad. 100,00% |
| TEGO® Alkanol 1618, Evonik Industries (INCI: Cetearyl Alcohol) | 1,00% |
| TEGO® Amid S 18, Evonik Industries (INCI: Stearamidopropyl Dimethylamine) | 1,20% |
| TEGIN® G 1100 Pellets, Evonik Industries (INCI: Glycol Distearate) | 0,30% |
| TEGO® Care PS, Evonik Industries (INCI: Methyl Glucose Sesquistearate) | 0,70% |
| TEGOSOFT® AC, Evonik Industries (INCI: Isoamyl Cocoate) | 0,20% |
| Synthesebeispiel 1.1 | 2,10% |
| Sodium Hydroxide (10% in water) | ad. pH 4,8 |
| Preservative, Perfume | q.s. |

**Formulierungsbeispiel 19) Leave-In Conditioner Spray**

| | |
|---|---|
| TAGAT® CH 40, Evonik Industries (INCI: PEG-40 Hydrogenated Castor Oil) | 2,50% |
| TEGO® Alkanol 1618, Evonik Industries (INCI: Cetearyl Alcohol) | 0,20% |
| Ceramide VI, Evonik Industries (INCI: Ceramide 6 II) | 0,05% |
| Perfume | 0,20% |
| Water | ad. 100,00% |
| Synthesebeispiel 1.6 | 2,50% |
| Argan Oil, DSM | 0,10% |
| LACTIL®, Evonik Industries (INCI: Sodium Lactate; Sodium PCA; Glycine; Fructose; Urea; Niacinamide; Inositol; Sodium benzoate; Lactic Acid) | 1,00% |
| TEGO® Betain F 50, Evonik Industries 38% (INCI: Cocamidopropyl Betaine) | 2,30% |
| Sodium Hydroxide (10% in water) | ad. pH 4,8 |

**Formulierunsbeisiel 20) Leave-In Conditioner Foam**

| | |
|---|---|
| Synthesebeispiel 1.1 | 1,50% |
| TAGAT® CH 40, Evonik Industries (INCI: PEG-40 Hydrogenated Castor Oil) | 0,50% |
| Perfume | 0,30% |
| TEGO® Betain 810, Evonik Industries (INCI: Capryl/Capramidopropyl Betaine) | 2,00% |
| Water | ad. 100,00% |
| TEGO® Cosmo C 100, Evonik Industries (INCI: Creatine) | 0,50% |
| TEGO® Alkanol 16, Evonik Industries (INCI: Cetyl Alcohol) | 0,20% |
| TEGOCEL® HPM 50, Evonik Industries (INCI: Hydroxypropyl Methylcellulose) | 0,30% |
| VARISOFT® 300, Evonik Industries (INCI: Cetrimonium Chloride) | 1,30% |
| LACTIL® Evonik Industries (INCI: Sodium Lactate; Sodium PCA; Glycine; Fructose; | 0,50% |
| Urea; Niacinamide; Inositol; Sodium benzoate; Lactic Acid) | |
| Citric Acid (30% in water) | ad. pH 4,0 |
| Preservative | q.s. |

**Formulierungsbeispiel 21) Strong Hold Styling Gel**

| | |
|---|---|
| TEGO® Carbomer 141, Evonik Industries (INCI: Carbomer) | 1,20% |
| Water | ad. 100,00% |
| NaOH, 25% | 2,70% |
| PVP/VA W-735, ISP (INCI: PVP/VA Copolymer) | 16,00% |
| Synthesebeispiel 1.2 | 1,50% |
| TEGO® Alkanol 16, Evonik Industries (INCI: Cetyl Alcohol) | 0,20% |
| Alcohol Denat. | 10,00% |
| TAGAT® O 2 V, Evonik Industries (INCI: PEG-20 Glyceryl Oleate) | 2,00% |
| Perfume | 0,30% |
| ABIL® B 88183 PH, Evonik Industries (INCI: PEG/PPG-20/6 Dimethicone) | 0,70% |
| Preservative | q.s. |

**Formulierungsbeispiel 22) Haarfärbemittel**

| | |
|---|---|
| Water demineralized | ad. 100,00% |
| TEGO® Alkanol 1618, Evonik Industries, (INCI: Cetearyl Alcohol) | 12,00% |
| Eutanol® G, BASF (INCI: Octyldodecanol) | 3,00% |
| REWOMID® C 212, Evonik Industries (INCI: Cocamide MEA) | 1,50% |
| Super Hartolan® B, Crodo (INCI: Lanolin Alcohol) | 3,00% |
| Avocadoöl, Henry Lamotte (INCI: Persea Gratissima Oil) | 1,50% |
| Pristerene® 4960, Uniquema (INCI: Stearic Acid) | 6,00% |
| EDTA BD, BASF (INCI: Disodium EDTA) | 0,10% |
| Texapon® K12G, BASF (INCI: Sodium Lauryl Sulfate) | 0,50% |
| Propylenglycol | 5,00% |
| Timica Silver Sparkle, BASF (INCI: MICA; Titanium Dioxide) | 1,00% |
| Ammoniaklösung, 25%ig | 6,00% |
| 2,5-Diaminotoluolsulfat, (INCI: Toluene-2,5-Diamine) | 1,40% |
| Rodol® RS, Jos. H. Lowenstein & Sons (INCI: Resorcinol) | 0,30% |
| HC Blue A42, (INCI: 2,4-Diaminophenoxyethanol di HCl) | 0,10% |
| Natriumsulfit | 0,50% |
| Perfume | 0,20% |
| Synthesebeispiel 1.1 | 0,50% |

**Formulierunsbeisiel 23) Haarfärbemittel**

| | |
|---|---|
| Water demineralized | ad. 100,00% |
| TEGO® Alkanol 1618, Evonik Industries (INCI: Cetearyl Alcohol) | 12,00% |
| Super Hartolan® B, Croda (INCI: Lanolin Alcohol) | 2,50% |
| Meadowfoam® Seed Oil, Fanning (INCI: Limnanthes Alba) | 1,00% |
| Pristerene® 4960, Uniquema (INCI: Stearic Acid) | 5,50% |
| EDTA BD, BASF (INCI: Disodium EDTA) | 0,10% |
| Glycerin | 5,00% |
| Texapon® N 70, BASF (INCI: Sodium Laureth Sulfate) | 2,00% |
| Monoethanolamin | 4,00% |
| 2,5-Diaminotoluolsulfat, (INCI: Toluene-2,5-Diamine) | 0,90% |
| Rodol® RS, Jos. H. Lowenstein & Sons (INCI: Resorcinol) | 0,20% |
| Jarocol® 4A3MP, Vivimed Labs (INCI: 4-Amino-M-Cresol) | 0,60% |
| Rodol® PAOC, Jos. H. Lowenstein & Sons (INCI: 4-Amino-2-Hydroxytoluene) | 0,50% |
| Uantox® EBATE, Universal Preserv-A-Chem (INCI: Erythorbic Acid) | 0,50% |
| Perfume | 0,20% |
| Synthesebeispiel 1.4 | 1,00% |

**Formulierungsbeispiel 24) Haarfärbemittel**

| | |
|---|---|
| Water demineralized | ad. 100,00% |
| TEGO® Alkanol 1618, Evonik Industries (INCI: Cetearyl Alcohol) | 10,00% |
| Eutanol® G, BASF Cognis (INCI: Octyldodecanol) | 1,00% |
| REWOMID® C 212, Evonik Industries (INCI: Cocamide MEA) | 2,00% |
| TEGIN® VS, Evonik Industries (INCI: Glyceryl Stearate SE) | 5,00% |
| Fitoderm®, Hispano Quimica S. A. (INCI: Squalane) | 1,00% |
| Coenzyme Q 10 | 0,10% |
| EDTA BD, BASF (INCI: Disodium EDTA) | 0,10% |
| Texapon® K12G, BASF (INCI: Sodium Lauryl Sulfate) | 0,10% |
| Propylenglycol | 5,00% |
| Ammoniaklösung, 25%ig | 3,00% |
| Rodol® ERN, Jos. H. Lowenstein & Sons (INCI: 1-Naphthol) | 0,30% |
| Imexine® OAG, Chimex (INCI: 2-Methyl-5-Hydroxyethylaminophenol) | 1,00% |
| Colorex® WP5, Teluca (INCI: 1-Hydroxyethyl 4,5-Diamino Pyrazole Sulfate) | 2,60% |
| Ascorbinsäure | 0,30% |
| Perfume | 0,30% |
| Synthesebeispiel 1.3 | 0,70% |

**Formulierungsbeispiel 25) Haarfärbemittel**

| | |
|---|---|
| Water demineralized | ad. 100,00% |
| TEGO® Alkanol 1618, Evonik Industries (INCI: Cetearyl Alcohol) | 13,00% |
| REWOMID® C 212, Evonik Industries (INCI: Cocamide MEA) | 2,00% |
| Super Hartolan® B, Croda (INCI: Lanolin Alcohol) | 2,50% |
| Avocadoöl, Henry Lamotte (INCI: Persea Gratissima Oil) | 2,00% |
| Pristerene® 4960, Uniquema (INCI: Stearic Acid) | 6,00% |
| EDTA BD, BASF (INCI: Disodium EDTA) | 0,10% |
| Texapon® K12G, BASF (INCI: Sodium Lauryl Sulfate) | 0,10% |
| Propylenglycol | 5,00% |
| Ammoniaklösung, 25%ig | 6,00% |
| 2,5-Diaminotoluolsulfat, (INCI: Toluene-2,5-Diamine) | 1,30% |
| Rodol® RS, Jos. H. Lowenstein & Sons (INCI: Resorcinol) | 0,30% |
| Covastyle® TBQ, LCW Les colorants Wackherr S.A. (INCI: t-Butyl Hydroquinone) | 0,30% |
| Perfume | 0,20% |
| Synthesebeispiel 1.5 | 0,50% |

## Patentansprüche

1. Wässrige Zusammensetzung enthaltend
A) ω-Aminocarbonsäureester und
B) Fettalkohol.

2. Zusammensetzung gemäß Anspruch 1, **dadurch gekennzeichnet, dass** Komponente A) ausgewählt ist aus ω-Aminocarbonsäureestem der allgemeinen Formel 1, der allgemeinen Formel 2 und der allgemeinen Formel 3, 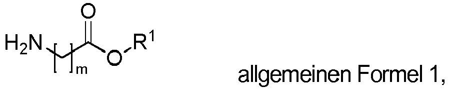 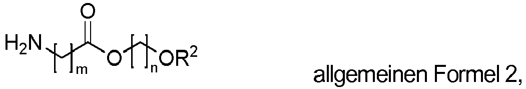 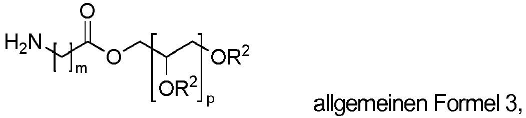 mit
m = 5 bis 21, bevorzugt 7 bis 17, besonders bevorzugt 11,
n = 2 bis 12, bevorzugt 3 bis 6,
p = 1 bis 10, bevorzugt 1 bis 4, besonders bevorzugt 1
R¹ = linearer oder verzweigter, gegebenenfalls ungesättigte Bindungen aufweisender Alkylrest mit 6 bis 22, bevorzugt 8 bis 18, besonders bevorzugt 12 bis 18, Kohlenstoffatomen,
R² = unabhängig voneinander H oder ein linearer oder verzweigter, gegebenenfalls ungesättigte Bindungen aufweisender Acylrest mit 6 bis 22, bevorzugt 8 bis 18, besonders bevorzugt 12 bis 18, Kohlenstoffatomen oder ein ω-Aminoacylrest, insbesondere

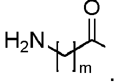

3. Zusammensetzung gemäß Anspruch 2, **dadurch gekennzeichnet, dass** Komponente A) ausgewählt ist aus
ω-Aminocarbonsäureestem der allgemeinen Formel 1 mit
m = 11 und
R¹ = ausgewählt aus Lauryl, Myristyl, Palmityl, Oleyl und Stearyl, und
ω-Aminocarbonsäureestem der allgemeinen Formel 3
m = 11, p = 1,
R² = ausgewählt aus H, Lauroyl, Myristoyl, Palmitoyl, Oleoyl, Stearoyl und 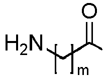 mit m = 11.

4. Zusammensetzung gemäß mindestens einem der vorherigen Ansprüche, **dadurch gekennzeichnet, dass** A) in einer Menge von 0,1 bis 7 Gew.-%, bevorzugt 0,2 bis 5 Gew.-% und insbesondere bevorzugt 0,3 bis 4 Gew.-%, wobei sich die Gew.-% auf die Gesamtzusammensetzung beziehen, enthalten ist.

5. Zusammensetzung gemäß mindestens einem der vorherigen Ansprüche zusätzlich enthaltend Anionen ausgewählt aus der Gruppe umfassend Chlorid, Bromid, lodid, Alkylsulfat, Alkylsulfonat, Triflat, Tosylat, Phosphat, Sulfat, Hydrogensulfat, Lactat, Glycolat, Acetat und Citrat.

6. Zusammensetzung gemäß mindestens einem der vorherigen Ansprüche, **dadurch gekennzeichnet, dass** B) in einer Menge von 0,5 bis 20 Gew.-%, bevorzugt 1 bis 10 Gew.-%, insbesondere 2 bis 7 Gew. %, in der erfindungsgemäßen Zusammensetzung, wobei sich die Gewichtsprozente auf die Gesamtzusammensetzung beziehen, enthalten ist.

7. Zusammensetzung gemäß mindestens einem der vorherigen Ansprüche zusätzlich enthaltend
C) Emulgator, insbesondere nicht-ionischen Emulgator.

8. Zusammensetzung gemäß Anspruch 7, **dadurch gekennzeichnet, dass** C) in einer Menge von 0,1 bis 10 Gew.-%, bevorzugt 0,25 bis 5 Gew.-%, insbesondere 0,5 bis 2,0 Gew.-%, in der erfindungsgemäßen Zusammensetzung, wobei sich die Gewichtsprozente auf die Gesamtzusammensetzung beziehen, enthalten ist.

9. Zusammensetzung gemäß mindestens einem der vorherigen Ansprüche aufweisend einen pH-Wert von 1 bis 6,9, besonders bevorzugt von 2 bis 6,5 insbesondere von 2,5 bis 6.

10. Verwendung eines ω-Aminocarbonsäureesters wie in einem der der Ansprüche 1 bis 3 genannt, sowie einer Zusammensetzung gemäß mindestens einem der Ansprüche 1 bis 9 zur kosmetischen Behandlung von Haut oder keratinischen Fasern.

11. Verwendung gemäß Anspruch 10 zur Erhöhung des Glanzes der Faser.

12. Verwendung gemäß Anspruch 10 zur Erhöhung der Flexibilität der Faser.

13. Verwendung gemäß Anspruch 10 zur Erhöhung der Kämmbarkeit der Faser.

14. Verwendung gemäß Anspruch 10 zur Erhöhung der Spannkraft der Faser.

15. Verwendung gemäß Anspruch 10 zur Reduktion der antistatischen Kräfte zwischen den Fasern.

16. Verwendung eines ω-Aminocarbonsäureesters wie in einem der der Ansprüche 1 bis 3 genannt als Emulgator.

## Claims

1. Aqueous composition comprising
A) ω-aminocarboxylic acid ester and
B) fatty alcohol.

2. Composition according to Claim 1, **characterized in that** component A) is selected from ω-aminocarboxylic acid esters of the general formula 1, the general formula 2 and the general formula 3, 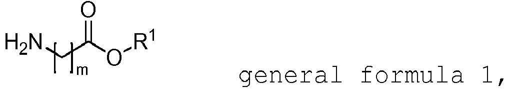 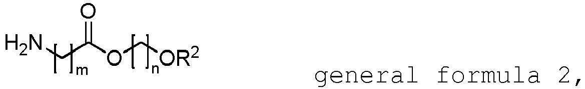 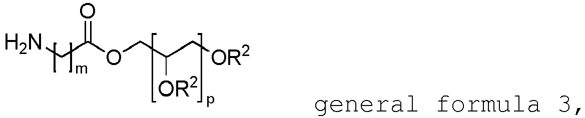 where
m = 5 to 21, preferably 7 to 17, particularly preferably 11,
n = 2 to 12, preferably 3 to 6,
p = 1 to 10, preferably 1 to 4, particularly preferably 1,
R¹ = linear or branched alkyl radical having 6 to 22, preferably 8 to 18, particularly preferably 12 to 18, carbon atoms and optionally having unsaturated bonds,
R² = independently of one another, H or a linear or branched acyl radical having 6 to 22,
preferably 8 to 18, particularly preferably 12 to 18, carbon atoms and optionally having unsaturated bonds, or an ω-aminoacyl radical, in particular

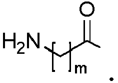

3. Composition according to Claim 2, **characterized in that** component A) is selected from
ω-aminocarboxylic acid esters of the general formula 1 where
m = 11 and
R¹ = selected from lauryl, myristyl, palmityl, oleyl and stearyl, and
ω-aminocarboxylic acid esters of the general formula 3
m = 11, p = 1,
R² = selected from H, lauroyl, myristoyl, palmitoyl, oleoyl, stearoyl and 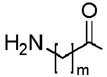 where m = 11.

4. Composition according to at least one of the preceding claims, **characterized in that** A) is present in an amount of from 0.1 to 7% by weight, preferably 0.2 to 5% by weight and particularly preferably 0.3 to 4% by weight, where the percentages by weight refer to the total composition.

5. Composition according to at least one of the preceding claims, additionally comprising anions selected from the group comprising chloride, bromide, iodide, alkyl sulphate, alkylsulphonate, triflate, tosylate, phosphate, sulphate, hydrogen sulphate, lactate, glycolate, acetate and citrate.

6. Composition according to at least one of the preceding claims, **characterized in that** B) is present in an amount of from 0.5 to 20% by weight, preferably 1 to 10% by weight, in particular 2 to 7% by weight, in the composition according to the invention, where the percentages by weight refer to the total composition.

7. Composition according to at least one of the preceding claims, additionally comprising
C) emulsifier, in particular nonionic emulsifier.

8. Composition according to Claim 7, **characterized in that** C) is present in an amount of from 0.1 to 10% by weight, preferably 0.25 to 5% by weight, in particular 0.5 to 2.0% by weight, in the composition according to the invention, where the percentages by weight refer to the total composition.

9. Composition according to at least one of the preceding claims, having a pH of from 1 to 6.9, particularly preferably from 2 to 6.5, in particular from 2.5 to 6.

10. Use of an ω-aminocarboxylic acid ester as specified in one of Claims 1 to 3, and of a composition according to at least one of Claims 1 to 9 for the cosmetic treatment of skin or keratin fibres.

11. Use according to Claim 10 for increasing the shine of the fibres.

12. Use according to Claim 10 for increasing the flexibility of the fibres.

13. Use according to Claim 10 for increasing the combability of the fibres.

14. Use according to Claim 10 for increasing the elasticity of the fibres.

15. Use according to Claim 10 for reducing the antistatic forces between the fibres.

16. Use of an ω-aminocarboxylic acid ester as specified in one of Claims 1 to 3 as emulsifier.

## Revendications

1. Composition aqueuse, contenant :
A) un ester d'acide ω-aminocarboxylique et
B) un alcool gras.

2. Composition selon la revendication 1, **caractérisée en ce que** le composant A) est choisi parmi les esters d'acides ω-aminocarboxyliques de formule générale 1, de formule générale 2 et de formule générale 3 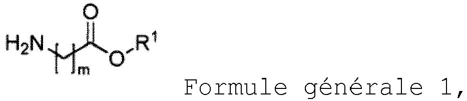 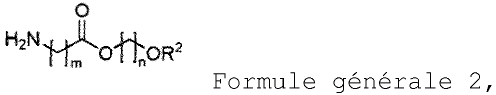 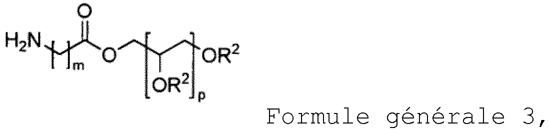 avec
m = 5 à 21, de préférence 7 à 17, de manière particulièrement préférée 11,
n = 2 à 12, de préférence 3 à 6,
p = 1 à 10, de préférence 1 à 4, de manière particulièrement préférée 1,
R¹ = radical alkyle linéaire ou ramifié, comprenant éventuellement des liaisons insaturées, de 6 à 22, de préférence 8 à 18, de manière particulièrement préférée 12 à 18, atomes de carbone,
les R² = indépendamment les uns des autres, H ou un radical acyle linéaire ou ramifié, comprenant éventuellement des liaisons insaturées, de 6 à 22, de préférence 8 à 18, de manière particulièrement préférée 12 à 18, atomes de carbone, ou un radical ω-aminoacyle, notamment

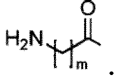

3. Composition selon la revendication 2, **caractérisée en ce que** le composant A) est choisi parmi :
les esters d'acides ω-aminocarboxyliques de formule générale 1 avec
m = 11 et
R¹ = choisi parmi lauryle, myristyle, palmityle, oléyle et stéaryle, et
les esters d'acides ω-aminocarboxyliques de formule générale 3 avec
m = 11, p = 1,
R² = choisi parmi H, lauroyle, myristoyle, palmitoyle, oléoyle, stéaroyle et 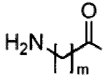 avec m = 11.

4. Composition selon au moins l'une quelconque des revendications précédentes, **caractérisée en ce que** A) est contenu en une quantité de 0,1 à 7 % en poids, de préférence de 0,2 à 5 % en poids et de manière particulièrement préférée de 0,3 à 4 % en poids, les % en poids se rapportant à la composition totale.

5. Composition selon au moins l'une quelconque des revendications précédentes, contenant en outre des anions choisis dans le groupe comprenant le chlorure, le bromure, l'iodure, l'alkylsulfate, l'alkylsulfonate, le triflate, le tosylate, le phosphate, le sulfate, l'hydrogénosulfate, le lactate, le glycolate, l'acétate et le citrate.

6. Composition selon au moins l'une quelconque des revendications précédentes, **caractérisée en ce que** B) est contenu en une quantité de 0,5 à 20 % en poids, de préférence de 1 à 10 % en poids, notamment de 2 à 7 % en poids, dans la composition selon l'invention, les % en poids se rapportant à la composition totale.

7. Composition selon au moins l'une quelconque des revendications précédentes, contenant en outre :
C) un émulsifiant, notamment un émulsifiant non ionique.

8. Composition selon la revendication 7, **caractérisée en ce que** C) est contenu en une quantité de 0,1 à 10 % en poids, de préférence de 0,25 à 5 % en poids, notamment de 0,5 à 2,0 % en poids, dans la composition selon l'invention, les % en poids se rapportant à la composition totale.

9. Composition selon au moins l'une quelconque des revendications précédentes, présentant un pH de 1 à 6,9, de manière particulièrement préférée de 2 à 6,5, notamment de 2,5 à 6.

10. Utilisation d'un ester d'acide ω-aminocarboxylique tel que mentionné dans l'une quelconque des revendications 1 à 3, ainsi que d'une composition selon au moins l'une quelconque des revendications 1 à 9, pour le traitement cosmétique de la peau ou de fibres kératiniques.

11. Utilisation selon la revendication 10 pour augmenter la brillance des fibres.

12. Utilisation selon la revendication 10 pour augmenter la flexibilité des fibres.

13. Utilisation selon la revendication 10 pour augmenter l'aptitude au peignage des fibres.

14. Utilisation selon la revendication 10 pour augmenter la tonicité des fibres.

15. Utilisation selon la revendication 10 pour réduire les forces antistatiques entre les fibres.

16. Utilisation d'un ester d'acide ω-aminocarboxylique tel que mentionné dans l'une quelconque des revendications 1 à 3 en tant qu'émulsifiant.
